# EUROPEAN PATENT APPLICATION

(11) **EP 4 520 746 A2**
(43) Date of publication of application: **12.03.2025**
(21) Application number: 25152670.3
(22) Date of filing: 08.09.2023
(51) Int. Cl.: C07C 55/10

(54) **SOLID FORMS OF A ROCK INHIBITOR**

(30) Priority: 08.09.2022 GB 202213103; 06.10.2022 GB 202214708
(62) Divisional of application: 23776436.0
(71) Applicant: Redx Pharma Limited, Macclesfield SK10 4TG (GB)
(72) Inventor: BELFIELD, Andrew, Macclesfield, SK10 4TG (GB); JONES, Clifford D, Macclesfield, SK10 4TG (GB); ARMER, Richard, Macclesfield, SK10 4TG (GB)
(74) Representative: HGF

(57) **Abstract**

This invention relates to a single enantiomer and crystalline forms of a Rho-associated protein kinase (ROCK) inhibitor, useful in the treatment of fibrotic diseases, e.g. fibrostenotic Crohn's disease.

## Description

This invention relates to a single enantiomer and crystalline forms of 4-(aminomethyl)-N-(3-fluoropyridin-4-yl)-3-{3-[(4-{[(2-oxooxolan-3-yl)methyl]sulfanyl}phenyl )carbamoyl]phenyl}benzamide (Compound A). Compound A is a Rho-associated protein kinase (ROCK) inhibitor, useful in the treatment of fibrotic diseases, e.g. fibrostenotic Crohn's disease.

### BACKGROUND

4-(aminomethyl)-N-(3-fluoropyridin-4-yl)-3-{3-[(4-{[(2-oxooxolan-3-yl)methyl]sulfan yl}phenyl)carbamoyl]phenyl}benzamide (Compound A) is an effective Rho-associated protein kinase (ROCK) inhibitor useful in the treatment of fibrotic diseases (see WO2014/118133). In particular Compound A has been designed to work specifically at the site of fibrosis in the gastrointestinal (GI) tract and to degrade quickly, if absorbed into the bloodstream, through enzyme-mediated metabolism.

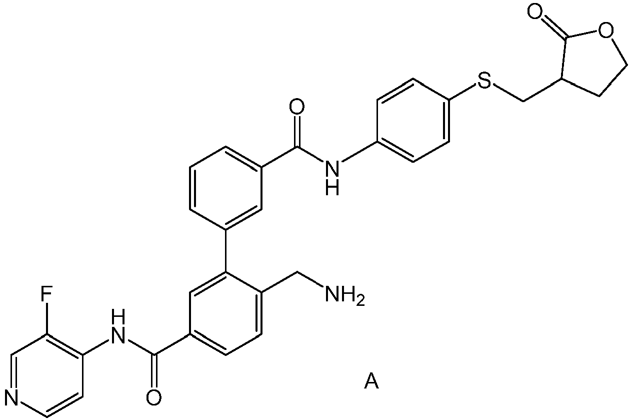

Crohn's disease is a chronic inflammatory bowel disease that affects 1.5m people globally and >70,000 new cases are diagnosed each year. Up to 50% of patients with Crohn's disease can develop significant fibrosis and stricture formation within ten years after diagnosis; this fibrosis associated with Crohn's disease is known as fibrostenotic Crohn's disease.

The current management of fibrotic strictures of the GI tract is primarily surgical as no drugs are specifically approved for fibrosis, which can progress despite intervention with anti-inflammatory therapies.

Relapse rate post surgical intervention is high with >50% patients requiring further surgery within 10 years, many within 12 months. Consequently, patients suffer progressive loss of G! function and repeated resections can lead to major health complications such as short bowel syndrome.

Preclinical data has shown that Compound A exhibits strong anti-fibrotic therapeutic effects in multiple animal models of inflammatory bowel disease.

The freebase of Compound A has low stability.

It is an aim of certain embodiments of this invention to provide a stable crystalline form of Compound A. It is an aim of certain embodiments of this invention to provide a crystalline form of Compound A that is more stable than other crystalline forms.

Certain embodiments of this invention satisfy some or all of the above aims.

### BRIEF SUMMARY OF THE DISCLOSURE

In a first aspect of the invention is provided the S-enantiomer (S-A) of compound A: 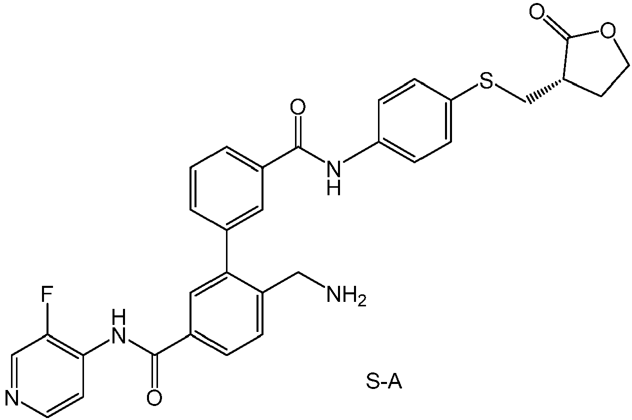 in substantially enantiopure form, or a pharmaceutically acceptable salt thereof. The S-enantiomer of compound A may be in the form of a freebase. The S-enantiomer of compound A may be in the form of a pharmaceutically acceptable salt. Said salt may be a crystalline salt form.

The inventors have found that the S-enantiomer of compound A exhibits lower inhibition of a wide-variety of alternative targets than the R-enantiomer. In particular, the S-enantiomer of compound A exhibits lower inhibition of a wide-variety of kinases other than ROCK than the R-enantiomer. This is particularly surprising as to date the inventors have detected no statistically meaningful difference between the activities of the S-enantiomer and the *R*-enantiomer of compound **A** against kinases ROCK1 or ROCK2.

In addition, the S-enantiomer is less active than the R-enantiomer in a significant number of assays that are used to identify undesirable off target activity that may cause adverse effects in humans.

Where the invention relates to a pharmaceutically acceptable salt of Compound A, the salt will typically be an acid addition salt. Suitable acid addition salts are formed from acids which form non-toxic salts. Examples include the acetate, aspartate, benzoate, besylate, bicarbonate/carbonate, bisulfate/sulfate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulfate, naphthylate, 1,5-naphthalenedisulfonate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

The salt may be selected from a hydrochloride salt and a succinate salt. The inventors have found that succinate and hydrochloride salts of compound A form crystals that are more stable than the freebase. The salt may be a succinate salt. The salt may be a hydrochloride salt. The inventors have found that crystalline forms of the hydrochloride salt of Compound A are particularly stable.

In a second aspect of the invention is provided a crystalline salt form of compound A:

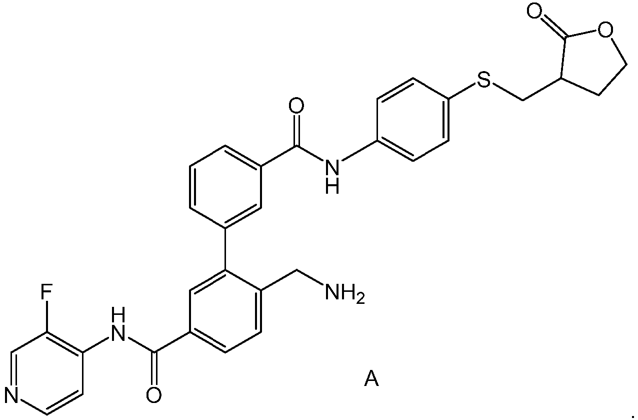

The freebase of Compound A has low chemical stability, particularly with respect to ester hydrolysis. The inventors have found that salt forms of Compound A exhibit improved stability relative to the freebase.

The invention relates to a pharmaceutically acceptable salt of Compound A. The salt will typically be an acid addition salt.

Suitable acid addition salts are formed from acids which form non-toxic salts. Examples include the acetate, aspartate, benzoate, besylate, bicarbonate/carbonate, bisulfate/sulfate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulfate, naphthylate, 1,5-naphthalenedisulfonate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

The salt may be selected from a hydrochloride salt and a succinate salt. The inventors have found that succinate and hydrochloride salts of compound A form crystals that are more stable than the freebase. The salt may be a succinate salt. The salt may be a hydrochloride salt. The inventors have found that crystalline forms of the hydrochloride salt of Compound A are particularly stable.

The crystalline salt form may be a solvate. The crystalline salt form may be a hydrate. The crystalline salt form may not be a solvate. The crystalline salt form may not be a hydrate.

Compound A contains a single chiral centre, at the point of attachment of the furanone ring to the rest of the molecule. In the crystal forms of the first aspect of the invention, Compound A may be a mixture of the two enantiomers. Compound A may be a racemic mixture of the two enantiomers. Compound A may be substantially in the form of a single enantiomer.

The single enantiomer may be the S-enantiomer (S-A):

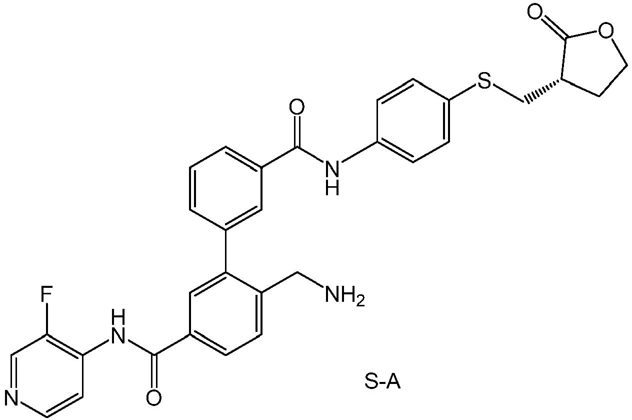

The single enantiomer may be the R-enantiomer (R-A):

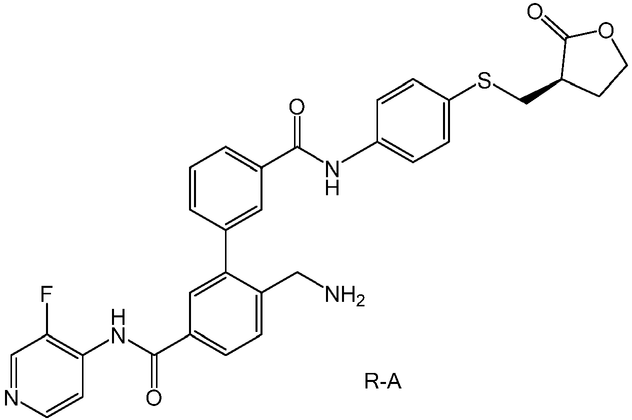

The word 'substantially' may mean that Compound A has an enantiomeric excess of 90% or greater. The word 'substantially' typically means that Compound A has an enantiomeric excess of 95% or greater. It may mean that Compound A has an enantiomeric excess of 98% or greater, 99% or greater or 99.5% or greater. Throughout this specification, where Compound A is described as being a single enantiomer (either R or S), it is intended to mean that it is in substantially enantiopure form within the meaning of this paragraph, unless an enantiomeric excess is otherwise specified.

The enantiomer may be the faster eluting enantiomer in chiral HPLC performed using a column that comprises silica coated in cellulose-tris(3,5-dimethylphenylcarbamate), e.g. a Chiralcel OD-3 column. The enantiomer may be the slower eluting enantiomer in chiral HPLC performed using a column that comprises silica coated in cellulose-tris(3,5-dimethylphenylcarbamate), e.g. a Chiralcel OD-3 column. The solvent system used as the mobile phase in the HPLC may be 28% solvent mixture B in solvent mixture A, wherein solvent mixture A is heptane containing 0.05% diethylamine; and solvent mixture B is a 4:1 mixture of isopropyl alcohol and acetonitrile, also containing 0.05% diethylamine.

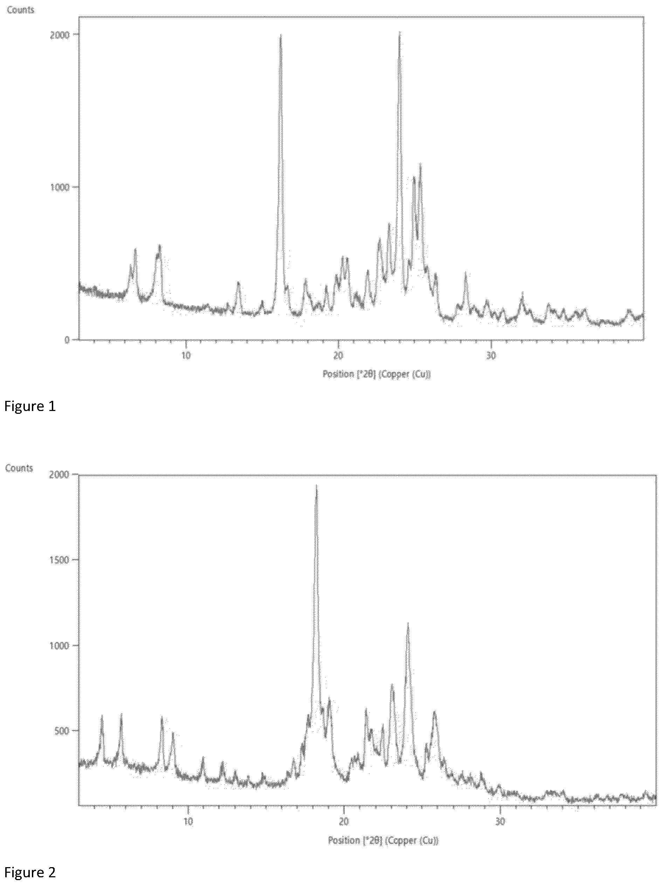

It may be that the salt is a hydrochloride salt of a single enantiomer of compound A, characterised in that said crystalline salt form has an XRPD pattern with at least two peaks at 2θ selected from 16.2± 0.2, 22.7± 0.2, 23.3± 0.2, 24.0± 0.2, 24.9± 0.2 and 25.4± 0.2 when measured using Cu radiation with a K_{α2} / K_{α1} ratio of 0.5. It may be that the salt is a hydrochloride salt of a single enantiomer of compound A, characterised in that said crystalline salt form has an XRPD pattern with at least four peaks at 2θ selected from 16.2± 0.2, 22.7± 0.2, 23.3± 0.2, 24.0± 0.2, 24.9± 0.2 and 25.4± 0.2 when measured using Cu radiation with a K_{α2} / K_{α1} ratio of 0.5. It may be that the salt is a hydrochloride salt of a single enantiomer of compound A, characterised in that said crystalline salt form has an XRPD pattern with peaks at 2θ selected from 16.2± 0.2, 22.7± 0.2, 23.3± 0.2, 24.0± 0.2, 24.9± 0.2 and 25.4± 0.2 when measured using Cu radiation with a K_{α2} / K_{α1} ratio of 0.5. It may be that said crystalline form has an XRPD pattern substantially as shown in Figure 1 when measured using Cu radiation with a K_{α2} / K_{α1} ratio of 0.5. This crystal form is known in this specification as HCl Salt Form I. This crystal form is a trihydrate. The single enantiomer may be the S enantiomer. The single enantiomer may be the R enantiomer.

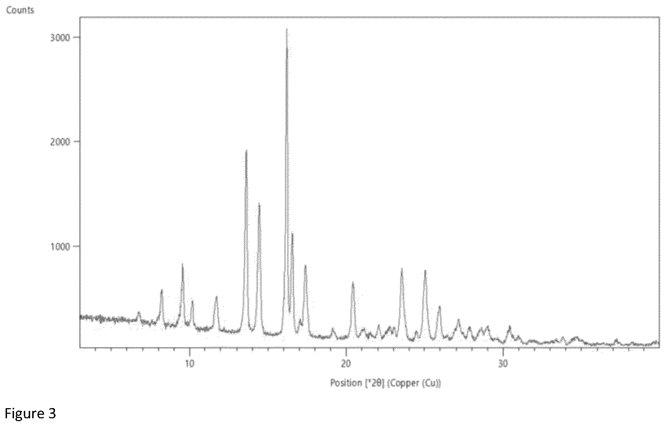

It may be that the salt is a hydrochloride salt of a single enantiomer of compound A, characterised in that said crystalline salt form has an XRPD pattern with at least two peaks at 2θ selected from 13.6± 0.2, 14.4± 0.2, 14.5± 0.2, 16.2± 0.2 and 16.5± 0.2 when measured using Cu radiation with a K_{α2} / K_{α1} ratio of 0.5. It may be that the salt is a hydrochloride salt of a single enantiomer of compound A, characterised in that said crystalline salt form has an XRPD pattern with at least four peaks at 2θ selected from 13.6± 0.2, 14.4± 0.2, 14.5± 0.2, 16.2± 0.2 and 16.5± 0.2 when measured using Cu radiation with a K_{α2} / K_{α1} ratio of 0.5. It may be that the salt is a hydrochloride salt of a single enantiomer of compound A, characterised in that said crystalline salt form has an XRPD pattern with peaks at 2θ selected from 13.6± 0.2, 14.4± 0.2, 14.5± 0.2, 16.2± 0.2 and 16.5± 0.2 when measured using Cu radiation with a K_{α2} / K_{α1} ratio of 0.5. It may be that said crystalline form has an XRPD pattern substantially as shown in Figure 3 when measured using Cu radiation with a K_{α2} / K_{α1} ratio of 0.5. This crystal form is known in this specification as HCl Salt Form II. This crystal form is an anhydrate. The single enantiomer may be the S enantiomer. The single enantiomer may be the R enantiomer.

It may be that the salt is a succinate salt of a single enantiomer of compound A, characterised in that said crystalline salt form has an XRPD pattern with at least two peaks at 2θ selected from 18.2± 0.2, 18.6± 0.2, 19.1± 0.2, 21.4± 0.2, 23.0± 0.2, 24.1± 0.2 and 25.8± 0.2 when measured using Cu radiation with a K_{α2} / K_{α1} ratio of 0.5. It may be that the salt is a succinate salt of a single enantiomer of compound A, characterised in that said crystalline salt form has an XRPD pattern with at least four peaks at 2θ selected from 18.2± 0.2, 18.6± 0.2, 19.1± 0.2, 21.4± 0.2, 23.0± 0.2, 24.1± 0.2 and 25.8± 0.2 when measured using Cu radiation with a K_{α2} / K_{α1} ratio of 0.5. It may be that the salt is a succinate salt of a single enantiomer of compound A, characterised in that said crystalline salt form has an XRPD pattern with peaks at 2θ selected from 18.2± 0.2, 18.6± 0.2, 19.1± 0.2, 21.4± 0.2, 23.0± 0.2, 24.1± 0.2 and 25.8± 0.2 when measured using Cu radiation with a K_{α2} / K_{α1} ratio of 0.5. It may be that said crystalline form has an XRPD pattern substantially as shown in Figure 2 when measured using Cu radiation with a K_{α2} / K_{α1} ratio of 0.5. This crystal form is known in this specification as Succinate Salt Form I. The single enantiomer may be the S enantiomer. The single enantiomer may be the R enantiomer.

HCl Salt Form I, HCl Salt Form II and Succinate Salt Form I are all chemically stable crystalline materials and are more stable than the freebase of compound A. HCl Salt Form I and HCl Salt Form II are more stable than Succinate Salt Form !. HCl Salt Form II is particularly beneficial as it is more crystalline than HCl Salt Form I and also more soluble at low pH.

In a third aspect of the invention is provided a pharmaceutical formulation comprising the crystalline salt form of the first aspect or the compound A, or pharmaceutical salt thereof, of the second aspect.

In a fourth aspect of the invention is provided the crystalline salt form of the first aspect or the compound A, or pharmaceutical salt thereof, of the second aspect for use in medical treatment.

In a fifth aspect of the invention is provided the crystalline salt form of the first aspect or the compound A, or pharmaceutical salt thereof, of the second aspect for use in treating a disease.

In a sixth aspect of the invention is provided a method of treating a disease, the method comprising administering to a subject in need thereof a therapeutically effective amount of the crystalline salt form of the first aspect or the compound A, or pharmaceutical salt thereof, of the second aspect.

In a seventh aspect of the invention is provided the use of the crystalline salt form of the first aspect or the compound A, or pharmaceutical salt thereof, of the second aspect in the manufacture of a medicament for treating a disease.

The disease may be a fibrotic disease.

The disease may be a disease of the gastrointestinal system.

The disease may be an inflammatory bowel disease.

The disease may be Crohn's disease, e.g. fibrostenotic Crohn's disease

The disease may be a cancer, e.g. colorectal cancer.

The disease may be a skin disease, e.g. keloids or scleroderma.

The disease may be an ocular disease.

In an eighth aspect of the invention is provided a method of making HCl crystalline salt form II of a single enantiomer of Compound A, the method comprising:
a) suspending a sample of HCl crystalline salt form I of a single enantiomer of Compound A in a solvent to form a suspension;
b) filtering the suspension to obtain HCl crystalline salt form II.

The solvent may be a mixture of solvents.

The solvent may be a polar aprotic organic solvent. The solvent may comprise a polar aprotic organic solvent. The solvent may be water. The solvent may comprise water. The solvent may be a polar protic organic solvent. The solvent may comprise a polar protic organic solvent.

The solvent may be a mixture of a polar aprotic organic solvent and a solvent selected from a polar protic organic solvent and water. The solvent may be a mixture of a polar aprotic organic solvent and water.

Suitable polar aprotic organic solvents include dimethylsulfoxide (DMSO), acetone, acetonitrile, ethers (e.g. THF, diglyme, ethylene glycol dimethyl ether, t-butylmethyl ether), esters (e.g. ethyl acetate).

Suitable polar protic organic solvents include ethanol, methanol, isopropanol, ethylene glycol.

The solvent may comprise acetone. The solvent is preferably a mixture of acetone and water. The inventors have found that use of a mixture of acetone and water provides a cleaner product than other possible solvent systems.

The proportion of polar aprotic organic solvent (e.g. acetone) to water may be in the range 1:1 to 50:1 acetone:water by volume. The proportion of polar aprotic organic solvent (e.g. acetone) to water may be in the range 10:1 to 30:1 acetone:water by volume. The proportion of polar aprotic organic solvent (e.g. acetone) to water may be in the range 15:1 to 25:1 acetone:water by volume.

The single enantiomer may be the S enantiomer. The single enantiomer may be the R enantiomer.

Step a) typically comprises stirring the mixture. The mixture may be stirred for greater than 1 h. The mixture may be stirred for greater than 5 h. The mixture may be stirred for greater than 12 h. The mixture may be stirred for less than 48 h. The mixture may be stirred for less than 24 h.

Step a) will typically be performed at a temperature in the range 5 °C to 40 °C. Step a) will typically be performed at a temperature in the range 15 °C to 30 °C.

The process may further comprise: step c) drying the HCl salt form II, e.g. under vacuum. Step c) may be carried out at a temperature in the range 30 °C to 60 °C. Step c) may be carried out for more than 30 min. Step c) may be carried out for less than 6 h, e.g. less than 3 h.

### BRIEF DESCRIPTION OF THE DRAWINGS

Embodiments of the invention are further described hereinafter with reference to the accompanying drawings, in which:
Figure 1 is an XRPD spectrum of HCl Salt Form I
Figure 2 is an XRPD spectrum of Succinate Salt Form I
Figure 3 is an XRPD spectrum of HCl Salt Form II

### DETAILED DESCRIPTION

The invention relates to pharmaceutically acceptable salts of Compound A. For a review on suitable salts, see "Handbook of Pharmaceutical Salts: Properties, Selection, and Use" by Stahl and Wermuth (Wiley-VCH, Weinheim, Germany, 2002).

Pharmaceutically acceptable acid addition salts of Compound A may be prepared by one or more of two methods:
by reacting Compound A with the desired acid;
by converting one salt of Compound A to another by reaction with an appropriate acid or base or by means of a suitable ion exchange column.

Such reactions are typically carried out in solution. The resulting salt may precipitate out and be collected by filtration or may be recovered by evaporation of the solvent. The degree of ionisation in the resulting salt may vary from completely ionised to almost non-ionised.

The term 'solvate' is used herein to describe a molecular complex comprising the compound of the invention and one or more pharmaceutically acceptable solvent molecules, for example, ethanol. The term 'hydrate' is employed when said solvent is water.

It is known in the art that an X-ray powder diffraction pattern may be obtained which has one or more measurement errors depending on measurement conditions (such as equipment, sample preparation or machine used). In particular, it is generally known that intensities in an X-ray powder diffraction pattern may fluctuate depending on measurement conditions and sample preparation. For example, persons skilled in the art of X-ray powder diffraction will realise that the relative intensities of peaks may vary according to the orientation of the sample under test and on the type and setting of the instrument used. The skilled person will also realise that the position of reflections can be affected by the precise height at which the sample sits in the diffractometer and the zero calibration of the diffractometer. The surface planarity of the sample may also have a small effect. Hence a person skilled in the art will appreciate that the diffraction pattern data presented herein is not to be construed as absolute and any crystalline form that provides a power diffraction pattern substantially identical to those disclosed herein fall within the scope of the present disclosure (for further information see Jenkins, R & Snyder, R.L. 'Introduction to X-Ray Powder Diffractometry' John Wiley & Sons, 1996)."

The term 'stable' may refer to chemical stability or physical stability. In particular freebase Compound A is chemically unstable whereas the crystal forms of the invention are chemically stable for up to 7 days at 40 °C and 75% relative humidity and/or are chemically stable for up to 7 days at 60 °C and ambient humidity. Crystal forms of the invention may be chemically stable for up to 3 months at 40 °C and 75% relative humidity and/or are chemically stable for up to 3 months at 25 °C and 60% relative humidity.

For the above-mentioned compounds of the invention the dosage administered will, of course, vary with the compound employed, the mode of administration, the treatment desired and the disorder indicated. For example, if the compound of the invention is administered orally, then the daily dosage of the compound of the invention may be in the range from 0.01 micrograms per kilogram body weight (µg/kg) to 100 milligrams per kilogram body weight (mg/kg).

A crystalline salt form or compound of the invention may be used on their own but will generally be administered in the form of a pharmaceutical composition in which the crystalline salt form or compound, or pharmaceutically acceptable salt thereof, of the invention is in association with a pharmaceutically acceptable adjuvant, diluent or carrier. Conventional procedures for the selection and preparation of suitable pharmaceutical formulations are described in, for example, "Pharmaceuticals - The Science of Dosage Form Designs", M. E. Aulton, Churchill Livingstone, 1988.

Depending on the mode of administration of the crystalline salt form or compound of the invention, the pharmaceutical composition which is used to administer the crystalline salt form or compound of the invention will preferably comprise from 0.05 to 99 %w (per cent by weight) crystalline salt form or compound of the invention, more preferably from 0.05 to 80 %w crystalline salt form or compound of the invention, still more preferably from 0.10 to 70 %w crystalline salt form or compound of the invention, and even more preferably from 0.10 to 50 % crystalline salt form or compound of the invention, all percentages by weight being based on total composition.

The pharmaceutical compositions may be administered topically (e.g. to the skin or eye) in the form, e.g., of creams, gels, lotions, solutions, suspensions, or systemically, e.g. by oral administration in the form of tablets, capsules, syrups, powders or granules; by rectal administration in the form of suppositories; or by inhalation in the form of an aerosol.

For oral administration the crystalline salt form or compound of the invention may be admixed with an adjuvant or a carrier, for example, lactose, saccharose, sorbitol, mannitol; a starch, for example, potato starch, corn starch or amylopectin; a cellulose derivative; a binder, for example, gelatine or polyvinylpyrrolidone; and/or a lubricant, for example, magnesium stearate, calcium stearate, polyethylene glycol, a wax, paraffin, and the like, and then compressed into tablets. If coated tablets are required, the cores, prepared as described above, may be coated with a concentrated sugar solution which may contain, for example, gum arabic, gelatine, talcum and titanium dioxide. Alternatively, the tablet may be coated with a suitable polymer dissolved in a readily volatile organic solvent.

For the preparation of soft gelatine capsules, the crystalline salt form or compound of the invention may be admixed with, for example, a vegetable oil or polyethylene glycol. Hard gelatine capsules may contain granules of the salt form using either the above-mentioned excipients for tablets. Also liquid or semisolid formulations of the crystalline salt form or compound of the invention may be filled into hard gelatine capsules. Liquid preparations for oral application may be in the form of syrups or suspensions, the balance being sugar and a mixture of ethanol, water, glycerol and propylene glycol. Optionally such liquid preparations may contain colouring agents, flavouring agents, sweetening agents (such as saccharine), preservative agents and/or carboxymethylcellulose as a thickening agent or other excipients known to those skilled in art.

The size of the dose for therapeutic purposes of crystalline salt form or compound of the invention will naturally vary according to the nature and severity of the conditions, the age and sex of the animal or patient and the route of administration, according to well-known principles of medicine.

Dosage levels, dose frequency, and treatment durations of crystalline salt form or compound of the invention are expected to differ depending on the formulation and clinical indication, age, and co-morbid medical conditions of the patient.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification, and the contents of all such papers and documents are incorporated herein by reference.

### EXAMPLES

### Example 1 - Formation of Racemic Compound 4

### Preparation of compound 3 -

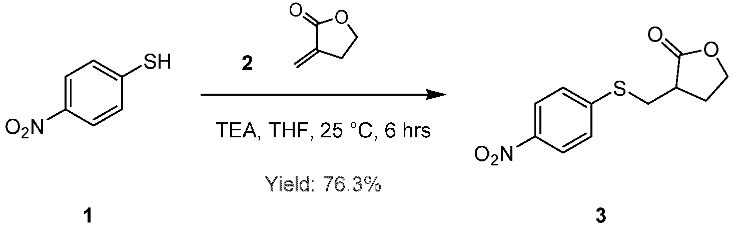

To a suspension of compound 2 (98.0 g, 999 mmol, 1.00 eq) in THF (tetrahydrofuran; 980 mL) was added TEA (triethylamine; 101 g, 999 mmol, 139 mL, 1.00 eq) and compound 1 (233 g, 1.50 mol, 1.50 eq) at 25 °C under N₂. The mixture was stirred at 25 °C under N₂ for 6 hrs. LCMS showed compound 2 was consumed and desired MS (RT = 0.592 min) was detected. The reaction was filtered and the solid was washed with petroleum ether (1.00 L) to wash off the dark colour. The filter cake was concentrated to give racemic compound 3 (389 g, 1.53 mol, 76.3% yield, 99.3% purity) as a yellow solid.

**LCMS:** product: RT = 0.592 min, m/z = 254.0 (M+H)⁺

**¹H NMR:** 400 MHz, DMSO *δ*/ppm: 8.15 (d, *J=* 8.8 Hz, 2H), 7.56 (d, *J* = 8.8 Hz, 2H), 4.35-4.30 (m, 1H), 4.20 - 4.15 (m, 1H), 3.54 (dd, *J* = 4.8, 4.4 Hz, 1H), 3.31 - 3.26 (m, 1H), 3.12 - 3.05 (m, 1H), 2.39 - 2.34 (m, 1H), 2.11 - 2.00 (m, 1H).

### Preparation of compound 4 -

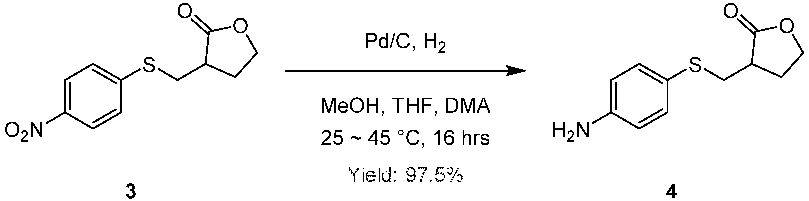

To a solution of racemic compound 3 (131 g, 512 mmol, 99.3% purity, 1.00 eq) in MeOH (366 mL), THF (366 mL) and DMA (dimethylacetamide; 52.0 mL) was Pd/C (41.8 g, 512 mmol, 10.0% purity, 1.00 eq) at 25 °C under N₂. The suspension was degassed and purged with H₂ (50 Psi) for three times. The mixture was heated to 45 °C and stirred under H₂ (50 Psi) at 45 °C for 16 hrs. TLC (Petroleum ether: Ethyl acetate = 1: 1) showed compound 3 (R_{f} = 0.60) was consumed and one new spot (R_{f} =0.30) was detected. Three batches of the reaction was filtered and concentrated in vacuum to leave only DMA which would not come off on the rotary evaporator. This was quenched into H₂O (1.00 L) and filtered, then the filter cake was concentrated to give compound 4 (336 g, 1.50 mol, 97.5% yield, 99.5% purity) as an off-white solid.

LCMS: product: RT = 0.260 min, m/z = 224.1 (M+H)⁺

HPLC: product: RT = 0.585 min, 99.5% purity under 220 nm.

SFC: (Racemate), Product: RT = 0.926 min, Product: Rt = 1.507 mins

**¹H NMR:** 400 MHz, DMSO *δ*/ppm: 7.13 (d, *J=* 8.8 Hz, 2H), 6.53 (d, *J=* 8.4 Hz, 2H), 5.29 (s, 2H), 4.31 - 4.26 (m, 1H), 4.17 - 4.11 (m, 1H), 3.08 (dd, *J* = 3.6, 3.6 Hz, 1H), 2.83 - 2.77 (m, 1H), 2.76 - 2.68 (m, 1H), 2.38 - 2.30 (m, 1H), 2.06 - 1.96 (m, 1H).

### Example 2 - Separation of enantiomers of compound 4

The two enantiomers of compound 4 were separated using super critical fluid chromatography (SFC) with a chiral column.

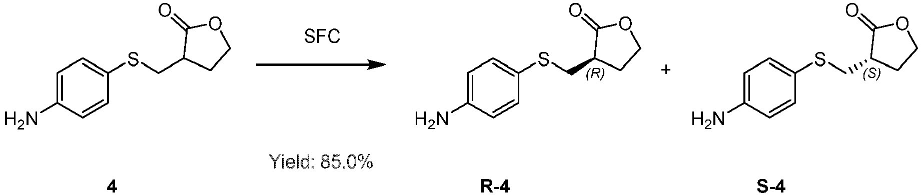

### SFC column conditions:

| | | |
|---|---|---|
| **Instrument** | Waters 350 Preparative SFC system | |
| **Column** | DaicelchiralpakAD column, 250×50mm I.D., 10um particle size; | |
| **Mobile Phase** | Phase A for Supercritical CO₂ | Phase B for MeOH |
| **Isocratic elution** | 50%Phase B ,50% Phase A | |
| **Flow rate** | 200g/min | |
| **cycle time** | 5.6 min | |
| **Back Pressure** | 100 bar to keep the CO2 in Supercritical flow | |
| **UV** | 220 nm | |

**Compound 4** (332 g, 1.48 mol, 99.5% purity) was purified by prep-SFC (column: DAICEL CHIRALPAK AD (250 mm * 50 mm, 10 um); mobile phase: [Neu - MeOH]; B%: 50% - 50%, 5.6; 2880 mins) to give **R-4** (150 g, 666 mmol, 45.0% yield, 99.1% purity) as yellow solid and **S-4** (162 g, crude) as yellow solid. The crude **S-4** was triturated with petroleum ether: ethyl acetate = 3: 1 (1.62 L) at 25 °C for 2 hrs to give **S-4** (132 g, 591 mmol, 40.0% yield, 99.8% purity) as a light yellow solid.

### R-4

**LCMS:** product: RT = 0.663 min, m/z = 224.0 (M+H)+
**HPLC:** product: RT = 1.419 mins, 99.1% purity under 220 nm.
**SFC:** (Homo chiral), Product: RT = 0.912 min, ee%: 99.4%
**¹HNMR:** 400 MHz, DMSO *δ*/ppm: 7.13 (d, *J=* 8.8 Hz, 2H), 6.53 (d, *J=* 8.4 Hz, 2H), 5.29 (s, 2H), 4.31 - 4.26 (m, 1H), 4.17 - 4.11 (m, 1H), 3.08 (dd, *J* = 3.6, 3.6 Hz, 1H), 2.83 - 2.77 (m, 1H), 2.76 - 2.68 (m, 1H), 2.38 - 2.30 (m, 1H), 2.06 - 1.96 (m, 1H)

### S-4

**LCMS:** product: RT = 0.656 min, m/z = 224.0 (M+H)⁺
**HPLC:** product: RT = 1.358 mins, 99.8% purity under 220 nm.
**SFC:** (Homo chiral), Product: RT = 1.491 mins, ee%: 99.7%
**¹H NMR:** 400 MHz, DMSO *δ*/ppm: 7.13 (d, *J=* 8.4 Hz, 2H), 6.53 (d, *J=* 8.8 Hz, 2H), 5.29 (s, 2H), 4.31 - 4.26 (m, 1H), 4.17 - 4.11 (m, 1H), 3.08 (dd, *J* = 3.6, 3.6 Hz, 1H), 2.83 - 2.77 (m, 1H), 2.75 - 2.68 (m, 1H), 2.38 - 2.30 (m, 1H), 2.06 - 1.96 (m, 1H)

### Example 3 - Conversion of R-4 into R-A

### Preparation of compound R-6

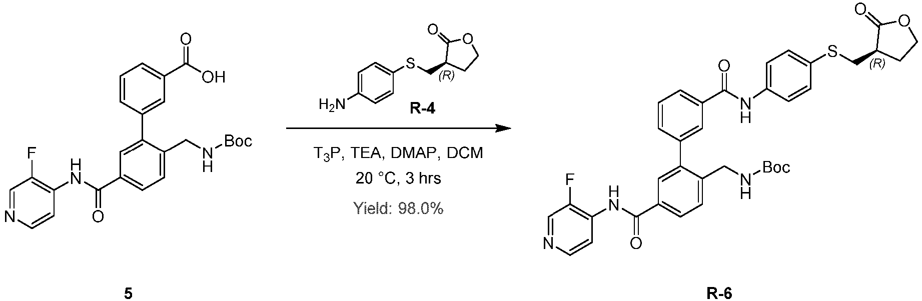

To a solution of **compound 5** (Boland et. al. Bioorg. Med. Chem. Lett. 2013, 6442-6446) (141 g, 294 mmol, 97.4% purity, 1.00 *eq*) in DCM (1.41 L) was added **compound R-4** (69.7 g, 309 mmol, 99.1% purity, 1.05 *eq*) and DMAP (dimethylaminopyridine; 7.19 g, 58.9 mmol, 0.200 *eq*), TEA (89.4 g, 883 mmol, 123 mL, 3.00 *eq*) at 20 °C under N₂. T₃P (Propylphosphonic anhydride; 281 g, 442 mmol, 263 mL, 50.0% purity, 1.50 eq) was added to the mixture at 20 °C and the mixture was stirred at 20 °C for 3 hrs. LCMS showed **compound 5** was consumed and desired MS (RT = 0.922 min) was detected. The reaction was diluted with DCM (1.40 L) and washed with saturated NaHCO₃ solution (1.40 L * 3). The organic layer was dried over Na₂SO₄, filtered and concentrated under reduced pressure to give a residue. The residue was purified by column chromatography (SiO₂, Petroleum ether: Ethyl acetate = 1: 0 to 0: 1, Petroleum ether: Ethyl acetate = 0: 1, R_{f} = 0.50) to give **compound R-6** (195 g, 289 mmol, 98.0% yield, 99.3% purity) as a light yellow solid.

LCMS: product: RT = 0.922 min, m/z = 671.3 (M+H)⁺

SFC: (Homo chiral), Product: RT = 4.269 mins, ee%: 100%

**¹H NMR:** 400 MHz, DMSO *δ*/ppm: 10.5 (s, 1H), 10.37 (s, 1H), 8.59 (d, *J=* 2.4 Hz, 1H), 8.39 (d, *J* = 5.6 Hz, 1H), 8.02 - 8.01 (m, 3H), 7.93 - 7.90 (m, 2H), 7.79 (d, *J* = 8.4 Hz, 2H), 7.70 - 7.64 (m, 2H), 7.57 (d, *J* = 8.0 Hz, 1H), 7.51 (t, *J* = 6.0 Hz, 1H), 7.42 (d, *J* = 8.8 Hz, 2H), 4.33 - 4.28 (m, 1H), 4.19 - 4.12 (m, 3H), 3.08 - 3.02 (m, 1H), 2.93 - 2.85 (m, 1H), 2.38 - 2.31 (m, 1H), 2.09 - 2.01 (m, 1H), 1.37 (s, 9H), 1.22 (s, 1H).

### Preparation of R-A

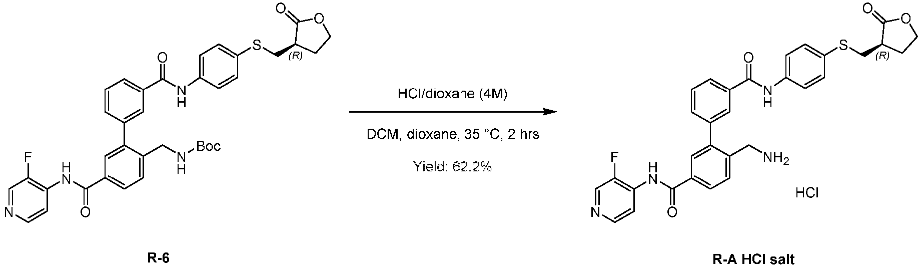

To a solution of compound R-6 (97.5 g, 144 mmol, 99.3% purity, 1.00 *eq*) in DCM (dichloromethane; 975 mL) and dioxane (975 mL) was added dropwise HCl/dioxane (4.00 M, 361 mL, 10.0 *eq*) at 35 °C. The mixture was stirred at 35 °C for 2 hrs. LCMS showed compound R-6 was consumed and desired MS (RT = 0.773 min) was detected. The reaction mixture was cooled to 25 °C and stirred at 25 °C for 12 hrs. The mixture was concentrated under reduced pressure to give a residue. The residue was diluted with deionized H₂O (2.00 L) and adjusted pH to 7 with sat. NaHCO₃ solution (300 mL), filtered and filter cake was concentrated. The crude product from both reactions was combined and triturated with deionized H₂O (1.50 L) at 25 °C for 12 hrs, then filtered and filter cake was concentrated to give R-A HCl (110 g, 180 mmol, 62.2% yield, 99.1% purity, HCl) as a light yellow solid.

**LCMS:** product: RT = 0.773 min, m/z = 571.3 (M+H)⁺

**HPLC:** product: RT = 7.942 mins, 99.1% purity under 220 nm.

**Chiral NP-HPLC,** Product: Rt = 13.573 mins, ee%: 100%

### Chiral NP-HPLC conditions:

| | | |
|---|---|---|
| **Instrument** | Agilent 1260 | |
| **Column** | Chiralcel OD-3 150×4.6mm I.D., 3µm particle size; | |
| **Mobile Phase** | Phase A : Heptane (0.05% DEA) | Phase B : IPA:CAN = 4:1 (0.05% DEA) |
| **Isocratic elution** | 28% Phase B in Phase A | |
| **Flow rate** | 1 mL/min | |
| **Column temp.** | 35°C | |
| **UV** | 220 nm | |

**EA-CHNS:** C: 54.0%, H: 5.36%, N: 7.98%, S: 4.59%

**¹H NMR:** 400 MHz, DMSO *δ*/ppm: 10.7 (s, 1H), 10.56 (s, 1H), 8.60 (d, *J=* 2.8 Hz, 1H), 5.54 (s, 2H), 8.40 (d, *J =* 5.2 Hz, 1H), 8.18 (s, 1H), 8.11 (dd, *J=* 2.0, 2.0 Hz, 1H), 8.07 - 8.05 (m, 2H), 7.91 - 7.88 (m, 2H), 7.85 (d, *J=* 8.8 Hz, 2H), 7.72 - 7.67 (m, 2H), 7.41 (d, *J =* 8.8 Hz, 2H), 4.33 - 4.28 (m, 1H), 4.18 - 4.12 (m, 1H), 4.06 (s, 2H), 3.32 (s, 1H), 3.08 - 3.02 (m, 1H), 2.91 - 2.87 (m, 1H), 2.38 - 2.31 (m, 1H), 2.09 - 2.02 (m, 1H).

### Example 4 - conversion of S-4 into S-A

### Preparation of compound S-6

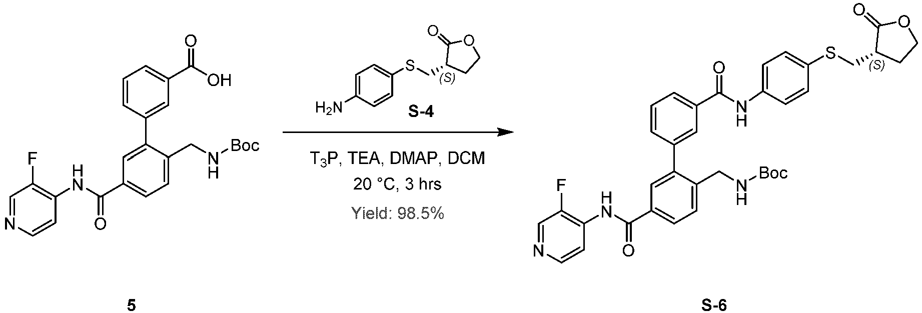

To a solution of **compound 5** (Boland et. al. Bioorg. Med. Chem. Lett. 2013, 6442-6446) (141 g, 294 mmol, 97.4% purity, 1.00 *eq*) in DCM (1.41 L) was added **compound S-4** (69.2 g, 309 mmol, 99.8% purity, 1.05 *eq*) and DMAP (7.19 g, 58.9 mmol, 0.200 eq), TEA (89.4 g, 883 mmol, 123 mL, 3.00 eq) at 20 °C under N₂. T₃P (281 g, 442 mmol, 263 mL, 50.0% purity, 1.50 eq) was added the mixture at 20 °C and the mixture was stirred at 20 °C for 3 hrs. LCMS showed **compound 5** was consumed and desired MS (RT = 0.917 min) was detected. The reaction was diluted with DCM (1.40 L) and washed with saturated NaHCO₃ solution (1.40 L * 3). The organic layer was dried over Na₂SO₄, filtered and concentrated under reduced pressure to give a residue. The residue was purified by column chromatography (SiO₂, Petroleum ether: Ethyl acetate = 1: 0 to 0: 1, Petroleum ether: Ethyl acetate = 0: 1, R_{f} = 0.50) to give **compound *S*-6** (197 g, 290 mmol, 98.5% yield, 98.9% purity) as a light yellow solid.

**LCMS:** product: RT = 0.917 min, m/z = 671.3 (M+H)⁺

**HPLC:** product: RT = 2.119 mins, 98.9% purity under 220 nm.

**SFC:** (Homo chiral), Product: Rt = 5.810 mins, ee%: 100%

**¹H NMR:** 400 MHz, DMSO *δ*/ppm: 10.5 (s, 1H), 10.4 (s, 1H), 8.59 (d, *J =* 2.4 Hz, 1H), 8.39 (d, *J* = 5.6 Hz, 1H), 8.02 - 8.01 (m, 3H), 7.93 - 7.90 (m, 2H), 7.79 (d, *J* = 8.8 Hz, 2H), 7.70 - 7.64 (m, 2H), 7.58 (d, *J* = 8.0 Hz, 1H), 7.51 (t, *J* = 6.0 Hz, 1H), 7.42 (d, *J* = 8.4 Hz, 2H), 4.33 - 4.28 (m, 1H), 4.19 - 4.12 (m, 3H), 3.08 - 3.02 (m, 1H), 2.93 - 2.85 (m, 1H), 2.38 - 2.32 (m, 1H), 2.09 - 2.01 (m, 1H), 1.38 (s, 9H), 1.22 (s, 1H).

### Preparation of S-A

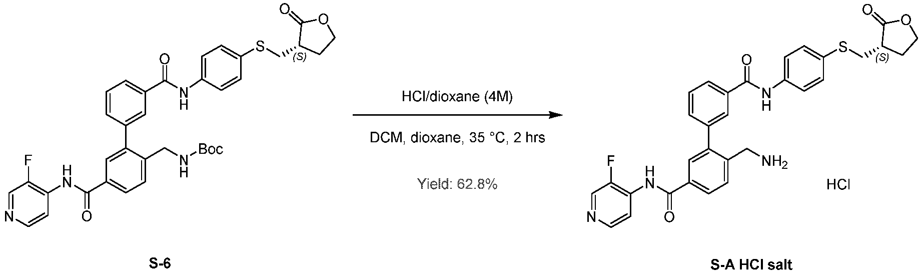

To a solution of compound S-6 (98.3 g, 145 mmol, 98.9% purity, 1.00 eq) in DCM (983 mL) and dioxane (983 mL) was added dropwise HCl/dioxane (4 M, 363 mL, 10.0 eq) at 35 °C. The mixture was stirred at 35 °C for 2 hrs. LCMS showed compound S-6 was consumed and desired MS (RT = 0.774 min) was detected. Two batches of the reaction mixture was cooled to 25 °C and stirred at 25 °C for 12 hrs. The mixture was concentrated under reduced pressure to give a residue. The residue was diluted with deionized H₂O (2.00 L) and adjusted pH to 7 with sat. NaHCO₃ solution (310 mL), filtered and filter cake was concentrated. The crude product from both reactions was combined and triturated with deionized H₂O (1.50 L) at 25 °C for 12 hrs, then filtered and filter cake was concentrated to give S-A HCl (112 g, 182 mmol, 62.8% yield, 98.8% purity, HCl) as a light yellow solid.

**LCMS:** product: RT = 0.774 min, m/z = 571.3 (M+H)⁺

**Chiral NP-HPLC,** Product: Rt = 11.407 mins, ee%: 100%

Chiral NP-HPLC are identical to those described in Example 3

**EA-CHNS:** C: 56.7%, H: 5.39%, N: 8.35%, S: 4.72%

**¹H NMR:** 400 MHz, DMSO *δ*/ppm: 10.7 (s, 1H), 10.5 (s, 1H), 8.60 (d, *J=* 2.8 Hz, 1H), 8.41 - 8.40 (m, 3H), 8.16 (s, 1H), 8.11 (dd, *J* = 2.0, 1.6 Hz, 1H), 8.08 - 8.06 (m, 2H), 7.91 - 7.87 (m, 2H), 7.85 (d, *J* = 8.8 Hz, 2H), 7.72 - 7.67 (m, 2H), 7.41 (d, *J* = 8.8 Hz, 2H), 4.33 - 4.28 (m, 1H), 4.18 - 4.12 (m, 1H), 4.06 (s, 2H), 3.36 (s, 1H), 3.08 - 3.02 (m, 1H), 2.91 - 2.87 (m, 1H), 2.37 - 2.31 (m, 1H), 2.07 - 2.02 (m, 1H).

This method directly forms *S*-**A** HCl Salt Form I. XRPD was carried out on *S*-**A** HCl Salt Form I using the following conditions:

### XRPD Conditions

XRPD diffractograms were collected with an X-ray diffractometer. The sample was prepared on a zero-background silicon wafer by gently pressing onto the flat surface. The parameters of XRPD diffraction are given below.

### Parameters of XRPD Testing

| | |
|---|---|
| **Instrument** | PANalytical, Empyrean |
| **Radiation** | Cu Kα (*λ* = 1.5418 Å) |
| **Detector** | PIXcel^{1D} |
| **Scan angle** | 3-40° (2θ) |
| **Scan step** | 0.013° (2θ) |
| **Scan speed** | 20.4 s/step |
| **Tube voltage/current** | 45 kV/40 mA |
| **Divergence slit** | 1/8° |
| **Rotation** | On |
| **Sample holder** | Zero-background sample pan |

### XRPD of S-A HCl Salt form I (see also Figure 1)

| Pos. [°2θ] | Rel. Int. [%] |
|---|---|
| 24.0066 | 100 |
| 16.2248 | 96 |
| 25.3699 | 53.78 |
| 24.9127 | 45.63 |
| 23.2962 | 32.79 |
| 22.7155 | 26.92 |
| 22.5886 | 23.04 |
| 20.2571 | 20.05 |
| 24.6041 | 19.95 |
| 20.5891 | 19.33 |

### Example 5 - Formation of of S-A Succinate Salt Form I

Formation of *S*-**A** Freebase Form I: Around 100 mg of *S*-**A** HCl Salt Form I was suspended in 1 mL of acetonitrile at room temperature (24 °C). A thick suspension was observed after stirring for 15 minutes, then 6 eq. of NaHCO₃ aqueous solution (84 mg of NaHCO₃ was dissolved in 0.8 mL of water) was added. After stirring for 35 minutes, a suspension was observed. And 2 mL of acetonitrile and 10 mL of water was added, then a solid mixed with oil was precipitated after stirring for 10 minutes. Finally, a suspension was obtained after stirring for 15h at RT. The solid was collected by filtration and dried at 40 °C under vacuum for ~3h to give *S*-**A** Freebase Form I.

Formation of *S*-**A** Succinate Form I: About 400 mg of *S*-**A** Freebase Form I and 1.1 eq. of succinic acid (91 mg) were added into 8 mL of MEK (methylethyl ketone) at room temperature (~24 °C). The mixture was stirred at room temperature for 4h, and then solid was collected by filtration and washed by MEK, and dried under vacuum condition at 40 °C for 24 h to provide *S*-**A** Succinate Salt Form I.

XRPD was carried out on *S*-**A** Succinate Salt Form !. Conditions were the same as those described in Example 4 for the XRPD analysis of HCl Salt form I.

### XRPD peaks of Succinate Salt form I (see also Figure 2)

| Pos. [°2θ] | Rel. Int. [%] |
|---|---|
| 18.2411 | 100 |
| 24.0587 | 54.67 |
| 22.9845 | 32.36 |
| 19.0701 | 28.48 |
| 25.781 | 27.21 |
| 18.6238 | 26.69 |
| 21.4017 | 26.55 |
| 22.4713 | 21.85 |
| 17.6381 | 21.12 |
| 25.9897 | 21.11 |

### Example 6 - formation of S-A HCl salt form II

About 600 mg of *S*-**A** HCl Salt Form I was suspended in 20 mL of acetone/water (19/1, v/v) at room temperature, and stirred for about 16 h. Solids were collected by filtration, dried under vacuum at 40 °C for ~2 h. Around 545 mg of HCl Salt Form II was prepared with yield of 91%.

XRPD was carried out on *S*-**A** HCl Salt Form II. Conditions were the same as those described in Example 4 for the XRPD analysis of HCl Salt form I.

XRPD peaks of *S*-**A** HCl Salt form II (see also Figure 3)

| Pos. [°2θ] | Rel. Int. [%] |
|---|---|
| 16.1933 | 100 |
| 13.6249 | 55.56 |
| 14.4085 | 39.56 |
| 14.48 | 37.81 |
| 16.5458 | 32.86 |
| 23.55 | 22.48 |
| 17.3667 | 22.34 |
| 25.053 | 21.46 |
| 9.5696 | 19.76 |
| 20.3741 | 16.16 |

### Example 7 - Stability Testing data

### Method:

About 10 mg of freebase Form I, HCl salt Form II, Succinate Form I and received HCl salt Form I were placed at 60 °C/capped and 40 °C/75% RH (open), respectively. Samples were prepared in duplicate for each condition. At day 0 and day 7, the samples were analyzed by HPLC and XRPD to check the purity and crystal form, respectively.

The results are provided in the following table.

| **Form** | **Conditions** | **Purity (area%)** | **XRPD** |
|---|---|---|---|
| Freebase Form I | Day 0 | 96.89 | N/A |
| | Day7-40 °C/75%RH | 93.87 **(3.0**↓**)** | No change |
| | Day7-60 °C/capped | 95.01 **(1.9**↓**)** | No change |
| HCl salt Form II | Day 0 | 98.65 | N/A |
| | Day7-40 °C/75%RH | 98.48 **(0.2**↓**)** | No change |
| | Day7-60 °C/capped | 98.45 **(0.2**↓**)** | No change |
| HCl salt Form I | Day 0 | 97.62 | N/A |
| | Day7-40 °C/75%RH | 97.62 | No change |
| | Day7-60 °C/capped | 97.61 | No change |
| Succinate Form I | Day 0 | 97.46 | N/A |
| | Day7-40 °C/75%RH | 97.29 **(0.2**↓**)** | No change |
| | Day7-60 °C/capped | 96.98 **(0.5**↓**)** | No change |

All three salt forms are more stable than freebase compound A. The two HCl salt forms are more stable than the succinate salt form.

The longer term stability on HCl salt form II was also tested under the following conditions:
- 25°C±2°C, 60% RH ± 5% RH
- 40°C±2°C, 75% RH ± 5% RH.

Samples were prepared in duplicate for each condition. At day 0 and at 1 month, and 3 months, the samples were analyzed by HPLC and XRPD to check the purity and crystal form, respectively.

The results are provided in the following tables.

### HCl Salt Form II Stability in Conditions 25°C±2°C, 60% RH ± 5% RH

| **Attribute** | **T0** | **T1 month** | **T3 month** |
|---|---|---|---|
| Appearance | White to off-white free-flowing solid, free from extraneous material | White to off-white free-flowing solid, free from extraneous material | White to off-white free-flowing solid, free from extraneous material |
| Assay by HPLC (water and solvent free) (area) | 101.6% | 101.4% | 101.2% |
| Total impurities by HPLC | Total impurities: 1.0% | Total impurities: 1.1% | Total impurities: 1.0% |
| Water Content | 1.8% | 1.6% | 1.4% |
| Polymorphism by XRPD | Form II | Form II | Form II |

HCl Salt Form II Stability in Conditions 40°C±2°C, 75% RH ± 5% RH

| **Attribute** | **T0** | **T1 month** | **T3 month** |
|---|---|---|---|
| Appearance | White to off-white free-flowing solid, free from extraneous material | White to off-white free-flowing solid, free from extraneous material | White to off-white free-flowing solid, free from extraneous material |
| Assay by HPLC (water and solvent free) (area) | 101.6% | 101.4% | 101.4% |
| Total impurities by HPLC | Total impurities: 1.0% | Total impurities: 1.1% | Total impurities: 1.1% |
| Water Content | 1.8% | 1.5% | 1.5% |
| Polymorphism by XRPD | **Form** II | **Form** II | **Form** II |

As can be seen, HCl salt form II is stable for up to 3 months even under accelerated conditions.

### Example 8 - Solubility Testing Data

### Method:

About 15 mg of sample was weighed into each sample vial and then 3.0 mL of water, simulated gastric fluid (SGF), Fasted State Simulated Intestinal Fluid (FaSSIF) or Fed State Simulated Intestinal Fluid (FeSSIF) media was added. Samples were prepared in triplicate for each media. The suspensions were kept shaking at 37 °C for up to 24h. At 0.5, 2 and 24h, suspensions were filtered and the filtrate was analyzed by HPLC.

The results are provided in the following tables.

| **Form** | **Media** | **pH of media** | **Solubility (mg/mL)** | | | **pH** | | |
|---|---|---|---|---|---|---|---|---|
| | | | **0.5 h** | **2h** | **24h** | **0.5 h** | **2h** | **24h** |
| HCl salt Form I | Water | 7.9 | 0.680 | 0.771 | 1.411 | 5.6 | 5.7 | 5.8 |
| | SGF | 1.2 | 0.780 | 0.841 | 1.355 | 1.4 | 1.4 | 1.4 |
| | FeSSIF | 5.0 | 0.083 | 0.091 | 0.066 | 4.9 | 4.9 | 4.9 |
| | FaSSIF | 6.5 | 0.023 | 0.005 | <LOD | 6.4 | 6.4 | 6.4 |
| HCl salt Form II | Water | 7.9 | 0.716 | 0.696 | 0.919 | 4.9 | 4.8 | 6.0 |
| | SGF | 1.2 | 2.550 | 2.591 | 3.372 | 1.2 | 1.4 | 1.4 |
| | FeSSIF | 5.0 | 0.064 | 0.069 | 0.057 | 4.9 | 4.9 | 4.9 |
| | FaSSIF | 6.5 | 0.019 | 0.023 | 0.006 | 6.4 | 6.4 | 6.4 |
| Succinate Form I | Water | 7.9 | 0.363 | 0.328 | 0.546 | 4.3 | 4.2 | 4.1 |
| | SGF | 1.2 | 3.837 | 3.826 | 4.005 | 1.3 | 1.4 | 1.3 |
| | FeSSIF | 5.0 | 0.188 | 0.173 | 0.046 | 4.8 | 4.8 | 4.8 |
| | FaSSIF | 6.5 | 0.005 | 0.002 | 0.001 | 5.7 | 5.7 | 5.6 |
| Freebase Form I | Water | 7.9 | <LOD | <LOD | <LOD | 7.9 | 7.6 | 8.7 |
| | SGF | 1.2 | ~5 | ~5 | ~5 | 1.2 | 1.4 | 1.4 |
| | FeSSIF | 5.0 | 0.183 | 0.126 | 0.059 | 5.0 | 5.0 | 5.0 |
| | FaSSIF | 6.5 | 0.014 | 0.006 | 0.001 | 6.4 | 6.4 | 6.5 |

HCl Salt Form II and Succinate Salt Form I were more soluble in SGF than HCl Salt Form I.

### Example 9 - Activity of R- and S-compound A against kinases other than ROCK

The HCl salts of S enantiomer and the R enantiomer of compound A were tested against a panel of kinases.

Compounds were received as powder and resuspended to 10 mM DMSO stock. Compounds were tested in 10-dose IC50 mode with a 3-fold serial dilution starting at 10 µM. Control compound, staurosporine, was tested in 10-dose IC50 mode with 4-fold serial dilution starting at 20 µM. Reactions were carried out at Km (Michaelis constant) ATP concentration (as indicated in the table).

Reaction Conditions: Buffer Conditions: 20 mM HEPES ((4-(2-hydroxyethyl)-1-piperazineethanesulfonic acid; pH 7.5), 10 mM MgCl₂, 1 mM EGTA (Ethyleneglycol- *bis*(β-aminoethyl)-N,N,N',N'-tetraacetic Acid), 0.01% Brij35, 0.02 mg/ml BSA (Bovine serum albumin), 0.1 mM Na₃VO₄, 2 mM DTT (Dithiothreitol), 1% DMSO. Reaction Procedure: 1. Prepare indicated substrate in freshly prepared reaction buffer. 2. Deliver required cofactors to the substrate solution above. 3. Deliver indicated kinase into the substrate solution and mix gently. 4. Deliver compounds in DMSO into the kinase reaction mixture utilizing acoustic technology (Echo550). 5. Deliver ³³P-ATP (specific activity 0.01 µCi/µl final) into the reaction mixture to initiate the reaction. 6. Incubate kinase reaction for 120 minutes at room temperature. 7. Reactions are spotted onto P81 ion exchange paper (Whatman # 3698-915). 8. Wash filters extensively in 0.75% phosphoric acid. 9. Measure the radioactive phosphorylated substrate remaining on the filter paper. Data Analysis: Kinase activity data were expressed as the percent remaining kinase activity in test samples compared to vehicle (dimethyl sulfoxide) reactions. IC50 values and curve fits were obtained using Prism4 Software (GraphPad).

The results are provided in the following table.

| | **Compound IC50 (nM)** | | **ATP Conc.** |
|---|---|---|---|
| **Kinase** | **R-enantiomer** | **S-enantiomer** | **(µM)** |
| **Haspin** | 134.4 | 297.1 | 20 |
| **LATS2** | 42.7 | 33.3 | 30 |
| **PKCd** | <0.5 | 0.9 | 10 |
| **PKCepsilon** | 2.9 | 6.7 | 10 |
| **PKCeta** | <0.5 | 0.9 | 15 |
| **PKCtheta** | 4.4 | 9.9 | 30 |
| **PKN1/PRK1** | 7.9 | 8.5 | 15 |

The S-enantiomer of compound **A** is less active than the *R*-enantiomer against six out of the seven off-target kinases. The *R*-enantiomer of compound **A** is less active than the *S-*enantiomer against just one out of the seven off-target kinases. This is particularly surprising as to date the inventors have detected no statistically meaningful difference between the activities of the *S*-enantiomer and the *R*-enantiomer of compound **A** against kinases ROCK1 or ROCK2.

### Example 10 - Activity of R- and S-compound A against CEREP Panel

The use of in vitro pharmacological screening against a diverse range of targets (receptors, ion channels, enzymes and transporters) is used to identify undesirable off-target activity that may cause adverse drug reactions in humans. Screening panels (e.g. SafetyScreen44^{™} Panel, Eurofins) comprise a selection of off-targets linked with known issues in humans that could hinder or halt the development of drug candidates or market withdrawal if discovered after a drug is approved. The HCl salts of the S enantiomer and the R enantiomer of compound A were tested against a panel of such targets.

Typical adverse drug reactions associated with these off-targets has been summarised (Bowes et al, Nature Reviews Drug Discovery 2012, 11, 909).

### G-Protein Coupled receptors:

Cannabinoid receptor CB1
Agonism/ activation: Euphoria and dysphoria; anxiety; memory impairment and poor concentration; analgesia; hypothermia
Antagonism: increased weight loss; emesis; depression
Cannabinoid receptor CB2
Antagonism: increase in inflammation; decrease in bone mass
Muscarinic acetylcholine receptor M1
Agonist: Proconvulsant; increase in gastric acid secretion; hypertension; tachycardia; hyperthermia
Antagonist: decrease in cognitive function; decrease in gastric acid secretion; blurred vision
Muscarinic acetylcholine receptor M2
Agonist: decrease in heart rate; reflex; increase in blood pressure; negative chronotropy and inotropy; decrease in cardiac conduction; decrease in cardiac action potential duration
Antagonist: Tachycardia; bronchoconstriction; tremors
µ-type opioid receptor (mu-MOP)
Agonist: Sedation; decrease in gastrointestinal motility; pupil constriction; abuse liability; respiratory depression; miosis; hypothermia
Antagonist: increase in gastrointestinal motility; dyspepsia; flatulence

### Enzymes

Monoamine oxidase A (MAO)
Inhibition: Increase in blood pressure when combined with amines such as tyramine; drug drug interaction potential; dizziness; sleep disturbances; nausea

### Transporters

Norepinephrine transporter uptake
Inhibition: Increase in heart rate; increase in blood pressure; increase in locomotor activity; constipation; abuse potential
Serotonin transporter uptake
Inhibition: Increase in gastrointestinal motility; decrease in upper gastrointestinal transit; decrease in plasma renin; increase in other serotonin-mediated effects; insomnia; anxiety; nausea; sexual dysfunction

The tests were carried out according to the methods described in the following tables and indicated literature references.

### CEREP Panel:

| **Assay** | **Source** | **Ligand** | **Conc.** | **Kd** | **Non Specific** | **Incubation** | **Detection Method** | **Ref.** |
|---|---|---|---|---|---|---|---|---|
| **MAO-A (antagonist radioligand)** | rat cerebral cortex | [3H]Ro 41-1049 | 10 nM | 14 nM | clorgyline (1 µM) | 60 min 37°C | Scintillation counting | 1 |

| **Assay** | **Source** | **Stimulus** | **Incubation** | **Measured Component** | **Detection Method** | **Ref.** |
|---|---|---|---|---|---|---|
| **Receptors** | | | | | | |
| **CB1 *(h)* (agonist effect)** | human recombina nt (CHO cells) | none (30 nM CP 55940 for control) | 30 min 37°C | cAMP (cyclic AMP) | HTRF (Homogen eous Time Resolved Fluoresce nce) | 2 |
| **CB1 *(h)* (antagonist effect)** | human recombina nt (Chinese Hamster Ovary (CHO) cells) | CP 55940 (1nM) | 30 min 37°C | cAMP | HTRF | 2 |
| **CB2 *(h)* (agonist effect)** | human recombina nt (CHO cells) | none (100 nM WIN 55212-2 for control) | 10 min 37°C | cAMP | HTRF | 2 |
| **CB2 *(h)* (antagonist effect)** | human recombina nt (CHO cells) | WIN 55212-2 (3nM) | 10 min 37°C | cAMP | HTRF | 2 |
| **M1 *(h)* (agonist effect)** | human recombina nt (CHO cells) | none (100 nM acetylcholine for control) | Room temperatur e | intracellular [Ca2+] | Fluorimetr y | 3 |
| **M1 *(h)* (antagonist effect)** | human recombina nt (CHO cells) | acetylcholine ( 10 nM ) | Room temperatur e | intracellular [Ca2+] | Fluorimetr y | 3 |
| **M2 *(h)* (agonist effect)** | human recombina nt (CHO cells) | none (3 µM acetylcholine for control) | 10 min 37°C | cAMP | HTRF | 4 |
| **M2 *(h)* (antagonist effect)** | human recombina nt (CHO cells) | acetylcholine (300 nM ) | 10 min 37°C | cAMP | HTRF | 4 |
| **µ (MOP) *(h)* (agonist effect)** | human recombina nt (CHO cells) | none (0.1 µM DAMGO ([D-Ala², *N-*MePhe⁴, Gly-ol]-enkephalin) for control) | 10 min 37°C | cAMP | HTRF | 5 |
| **µ (MOP) *(h)* (antagonist effect)** | human recombina nt (CHO cells) | DAMGO (10 nM ) | 10 min 37°C | cAMP | HTRF | 5 |

| **Transporters** | | | | | | |
|---|---|---|---|---|---|---|
| **Norepinephri ne transporter uptake (h)** | human recombina nt | norepinephri ne hydrochlorid e, DL-[7-3H(N)] (500 nM) | 120 min Room temp | [3H]NE incorporation into cells | Scintillatio n counting | 6 |
| Serotonin **transporter uptake (h)** | human recombina nt | 5HT (150 nM) / 3H 5HT | 180 min Room temp | [3H]5HT incorporation in cells | Scintillatio n counting | 7 |

| | | | | | | |
|---|---|---|---|---|---|---|
| **1.** Cesura, A.M. et al. (1990), Mol. Pharmacol., 37: 358-366. **2.** Felder, C.C. et al. (1995), Mol. Pharmacol., 48: 443-450. **3.** Sur, C. et al. (2003), Proc. Natl. Acad. Sci. U.S.A., 100: 13674-13679. **4.** Michal, P. et al. (2001), Brit. J. Pharmacol., 132: 1217-1228. **5.** Wang, J.B. et al. (1994), FEBS Lett., 338: 217-222. **6.** Perovic, S. and Muller, W.E.G. (1995), Arzneim-Forsch. Drug Res., 45: 1145-1148. **7.** Verrico C. et al. (2007), Psychopharmacology, 189, 489-503. | | | | | | |

The results are provided in the following table.

| | **R-enantiomer** | **S-enantiomer** |
|---|---|---|
| **CB1 (h) (agonist effect) (EC₅₀)** | Inactive | Inactive |
| **CB1 (h) (antagonist effect) (IC₅₀)** | Inactive | Inactive |
| **CB2 (h) (agonist effect) (EC₅₀)** | Inactive | Inactive |
| **CB2 (h) (antagonist effect) (IC₅₀)** | 38µM | Inactive |
| **M1 (h) (agonist effect) (EC₅₀)** | Inactive | Inactive |
| **M1 (h) (antagonist effect) (IC₅₀)** | 0.77µM | >30µM |
| **M2 (h) (agonist effect) (EC₅₀)** | >10µM | 93µM |
| **M2 (h) (antagonist effect) (IC₅₀)** | 6.2µM | >100µM |
| **MAO-A (antagonist radioligand) (IC₅₀)** | 4.3µM | 0.82µM |
| **mu (MOP) (h) (agonist effect) (EC₅₀)** | 3.0µM | 84µM |
| **mu (MOP) (h) (antagonist effect) (IC₅₀)** | Inactive | Inactive |
| **Norepinephrine transporter uptake (h) (IC₅₀)** | 8.9µM | 21µM |
| **Serotonin transporter uptake (h) (IC₅₀)** | 6.0µM | 11.0µM |

The *S*-enantiomer of compound **A** is less active than the *R*-enantiomer in seven out of the thirteen assays. The *R*-enantiomer of compound **A** is less active than the *S*-enantiomer in just one of the thirteen assays.

The combination of this data against the CEREP panel and the data provided in Example 9 against the kinase panel indicates that the S-enantiomer is more selective than the R-enantiomer against a diverse range of off-target proteins and is less likely to be associated with undesirable toxicities and side effects that limit efficacy and tolerability in humans.

The invention may be as described in any of the following numbered clauses:
1. The S-enantiomer (S-A) of compound A: 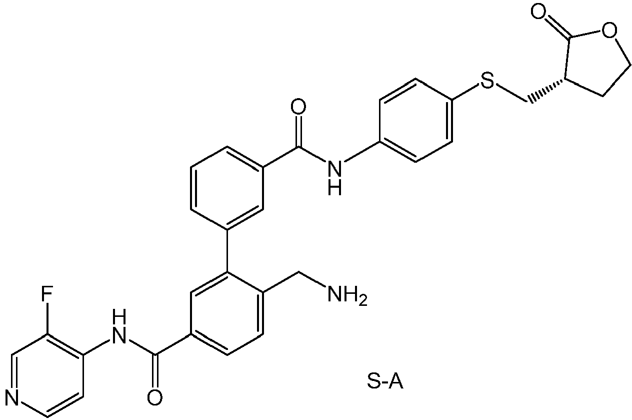 in substantially enantiopure form, or a pharmaceutically acceptable salt thereof.
2. The compound A of clause 1, wherein the compound A is in the form of a freebase.
3. The compound A of clause 1, wherein the compound A is in the form of a pharmaceutically acceptable salt.
4. A crystalline salt form of compound A: 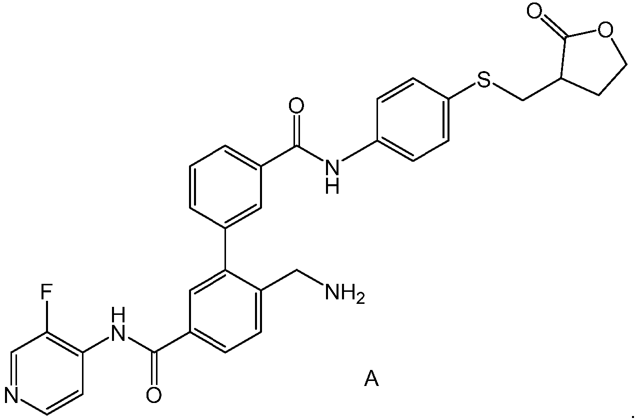
5. The crystalline salt form of clause 4, wherein the salt is a hydrochloride salt.
6. The crystalline salt form of clause 4, wherein the salt is a succinate salt.
7. The crystalline salt form of any one of clauses 4 to 6, wherein compound A is racemic.
8. The crystalline salt form of any one of clauses 4 to 6, wherein compound A is substantially in the form of a single enantiomer.
9. The crystalline salt form of clause 8, wherein the enantiomer is the S-enantiomer (S-A): 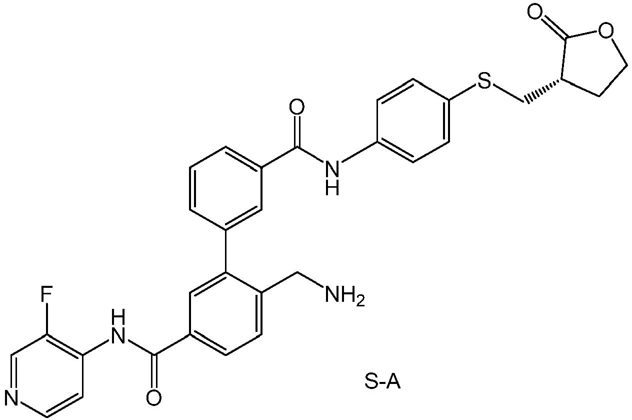
10. The crystalline salt form of clause 8, wherein the enantiomer is the R-enantiomer (R-A): 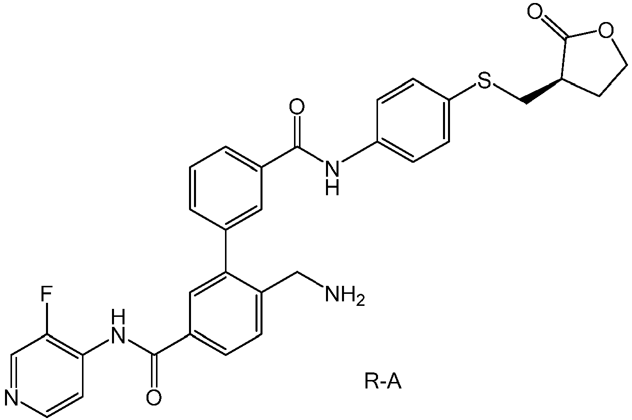
11. The crystalline salt form of clause 5, wherein the salt is a hydrochloride salt of a single enantiomer of compound A, characterised in that said crystalline salt form has an XRPD pattern with at least two peaks at 2θ selected from 16.2± 0.2, 22.7± 0.2, 23.3± 0.2, 24.0± 0.2, 24.9± 0.2 and 25.4± 0.2 when measured using Cu radiation with a K_{α2} / K_{α1} ratio of 0.5.
12. The crystalline salt form of clause 11, characterised in that said crystalline salt form has an XRPD pattern with peaks at 2θ selected from 16.2± 0.2, 22.7± 0.2, 23.3± 0.2, 24.0± 0.2, 24.9± 0.2 and 25.4± 0.2 when measured using Cu radiation with a K_{α2} / K_{α1} ratio of 0.5.
13. The crystalline salt form of clause 11, characterised in that said crystalline form has an XRPD pattern substantially as shown in Figure 3 when measured using Cu radiation with a K_{α2} / K_{α1} ratio of 0.5.
14. The crystalline salt form of clause 5, wherein the salt is a hydrochloride salt of a single enantiomer of compound A, characterised in that said crystalline salt form has an XRPD pattern with at least two peaks at 2θ selected from 13.6± 0.2, 14.4± 0.2, 14.5± 0.2, 16.2± 0.2 and 16.5± 0.2 when measured using Cu radiation with a K_{α2} / K_{α1} ratio of 0.5.
15. The crystalline salt form of clause 14, characterised in that said crystalline salt form has an XRPD pattern with peaks at 2θ selected from 13.6± 0.2, 14.4± 0.2, 14.5± 0.2, 16.2± 0.2 and 16.5± 0.2 when measured using Cu radiation with a K_{α2} / K_{α1} ratio of 0.5.
16. The crystalline salt form of clause 14, characterised in that said crystalline form has an XRPD pattern substantially as shown in Figure 1 when measured using Cu radiation with a K_{α2} / K_{α1} ratio of 0.5.
17. The crystalline salt form of clause 6, wherein the salt is a succinate salt of a single enantiomer of compound A, characterised in that said crystalline salt form has an XRPD pattern with at least two peaks at 2θ selected from 18.2± 0.2, 18.6± 0.2, 19.1± 0.2, 21.4± 0.2, 23.0± 0.2, 24.1± 0.2 and 25.8± 0.2 when measured using Cu radiation with a K_{α2} / K_{α1} ratio of 0.5.
18. The crystalline salt form of clause 17, characterised in that said crystalline salt form has an XRPD pattern with peaks at 2θ selected from 18.2± 0.2, 18.6± 0.2, 19.1± 0.2, 21.4± 0.2, 23.0± 0.2, 24.1± 0.2 and 25.8± 0.2 when measured using Cu radiation with a K_{α2} / K_{α1} ratio of 0.5.
19. The crystalline salt form of clause 17, characterised in that said crystalline form has an XRPD pattern substantially as shown in Figure 2 when measured using Cu radiation with a K_{α2} / K_{α1} ratio of 0.5.
20. A pharmaceutical composition comprising the compound A of any one of clauses 1 to 3 or of the crystalline salt form of any one of clauses 4 to 19.
21. The compound A of any one of clauses 1 to 3 or the crystalline form of any one of clauses 4 to 19 or for treating a fibrotic disease.
22. A salt form for use of clause 21, wherein the fibrotic disease is a disease of the gastrointestinal system.
23. A salt form for use of clause 21, wherein the fibrotic disease is fibrostenotic Crohn's disease.
24. A method of making HCl crystalline salt form II of a single enantiomer of Compound A, the method comprising:
   a) suspending a sample of HCl crystalline salt form I of a single enantiomer of Compound A in a solvent to form a suspension;
   b) filtering the suspension to obtain HCl crystalline salt form II.
25. A method of clause 24, wherein the solvent is a mixture of acetone and water.

## Claims

1. The S-enantiomer (S-A) of compound A: 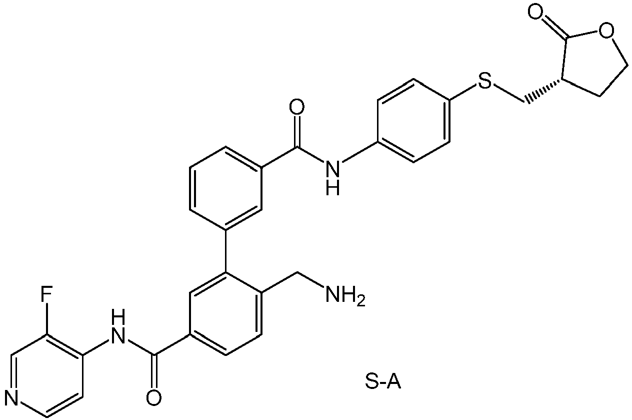 having an enantiomeric excess of 90% or greater, or a pharmaceutically acceptable salt thereof.

2. The compound A of claim 1, wherein the compound A is in the form of a freebase.

3. The compound A of claim 1, wherein the compound A is in the form of a pharmaceutically acceptable salt.

4. The compound A of claim 3, wherein the compound A is in a crystalline salt form.

5. The crystalline salt form of claim 4, wherein the salt is a hydrochloride salt.

6. The crystalline salt form of claim 4, wherein the salt is a succinate salt.

7. The crystalline salt form of claim 5, wherein the salt is a hydrochloride salt of a single enantiomer of compound A, **characterised in that** said crystalline salt form has an XRPD pattern with at least two peaks at 2θ selected from 16.2± 0.2, 22.7± 0.2, 23.3± 0.2, 24.0± 0.2, 24.9± 0.2 and 25.4± 0.2 when measured using Cu radiation with a K_{α2} / K_{α1} ratio of 0.5; optionally wherein the crystalline salt form is **characterised in that** said crystalline salt form has an XRPD pattern with peaks at 2θ 16.2± 0.2, 22.7± 0.2, 23.3± 0.2, 24.0± 0.2, 24.9± 0.2 and 25.4± 0.2 when measured using Cu radiation with a K_{α2} / K_{α1} ratio of 0.5.

8. The crystalline salt form of claim 7, **characterised in that** said crystalline form has an XRPD pattern substantially as shown in Figure 1 when measured using Cu radiation with a K_{α2} / K_{α1} ratio of 0.5.

9. The crystalline salt form of claim 5, wherein the salt is a hydrochloride salt of a single enantiomer of compound A, **characterised in that** said crystalline salt form has an XRPD pattern with at least two peaks at 2θ selected from 13.6± 0.2, 14.4± 0.2, 14.5± 0.2, 16.2± 0.2 and 16.5± 0.2 when measured using Cu radiation with a K_{α2} / K_{α1} ratio of 0.5; optionally wherein the crystalline salt form is **characterised in that** said crystalline salt form has an XRPD pattern with peaks at 2θ 13.6± 0.2, 14.4± 0.2, 14.5± 0.2, 16.2± 0.2 and 16.5± 0.2 when measured using Cu radiation with a K_{α2} / K_{α1} ratio of 0.5.

10. The crystalline salt form of claim 9, **characterised in that** said crystalline form has an XRPD pattern substantially as shown in Figure 3 when measured using Cu radiation with a K_{α2} / K_{α1} ratio of 0.5.

11. The crystalline salt form of claim 6, wherein the salt is a succinate salt of a single enantiomer of compound A, **characterised in that** said crystalline salt form has an XRPD pattern with at least two peaks at 2θ selected from 18.2± 0.2, 18.6± 0.2, 19.1± 0.2, 21.4± 0.2, 23.0± 0.2, 24.1± 0.2 and 25.8± 0.2 when measured using Cu radiation with a K_{α2} / K_{α1} ratio of 0.5; optionally wherein the crystalline salt form is **characterised in that** said crystalline salt form has an XRPD pattern with peaks at 2θ 18.2± 0.2, 18.6± 0.2, 19.1± 0.2, 21.4± 0.2, 23.0± 0.2, 24.1± 0.2 and 25.8± 0.2 when measured using Cu radiation with a K_{α2} / K_{α1} ratio of 0.5.

12. The crystalline salt form of claim 11, **characterised in that** said crystalline form has an XRPD pattern substantially as shown in Figure 2 when measured using Cu radiation with a K_{α2} / K_{α1} ratio of 0.5.

13. A pharmaceutical composition comprising the compound A of any one of claims 1 to 12.

14. The compound A of any one of claims 1 to 12 for use in treating a fibrotic disease.

15. The compound A for use of claim 14, wherein the fibrotic disease is a disease of the gastrointestinal system; optionally wherein the fibrotic disease is fibrostenotic Crohn's disease.
